Europäisches Patentamt

(19) European Patent Office

Office européen des brevets

(11) Numéro de publication : **0 295 169 B1**

(12) **FASCICULE DE BREVET EUROPEEN**

(45) Date de publication du fascicule du brevet :
08.05.91 Bulletin 91/19

(51) Int. Cl.⁵ : **F17C 7/00**

(21) Numéro de dépôt : **88401298.0**

(22) Date de dépôt : **27.05.88**

(54) **Procédé et équipement pour fournir du CO2 supercritique.**

(30) Priorité : 05.06.87 FR 8707901

(43) Date de publication de la demande :
14.12.88 Bulletin 88/50

(45) Mention de la délivrance du brevet :
08.05.91 Bulletin 91/19

(84) Etats contractants désignés :
BE CH DE ES IT LI NL SE

(56) Documents cités :
FR-A- 1 259 151
GB-A- 621 019
US-A- 3 216 209

(73) Titulaire : **CARBOXYQUE FRANCAISE**
**Tour Générale 5, place de la Pyramide**
**F-92800 Puteaux (FR)**

(72) Inventeur : **Buiguez, Alexandre**
**559, Route Nationale Vallon des Vaux**
**F-13400 Aubagne (FR)**
Inventeur : **Percy du Sert, Michel**
**48, bd Murat**
**F-75016 Paris (FR)**
Inventeur : **Frejaville, Serge**
**Claud du Genais**
**F-30340 Mejannes-les-Ales (FR)**

(74) Mandataire : **Jacobson, Claude et al**
**Cabinet Lavoix 2, Place d'Estienne d'Orves**
**F-75441 Paris Cedex 09 (FR)**

## Description

La présente invention est relative à un procédé pour fournir de l'anhydride carbonique supercritique, utilisable par exemple dans l'industrie cosmétique en tant qu'agent solvant et propulseur.

La technique courante pour fournir du $CO_2$ supercritique consiste à utiliser un stockage de $CO_2$ liquide à –20°C, 20 bars, une pompe haute pression à plusieurs étages et un groupe frigorifique, ce qui est coûteux et compliqué.

L'invention a pour but de fournir un procédé beaucoup plus simple et économique.

Un tel procédé constitue l'objet de la revendication 1.

Suivant le mode avantageux de remplissage du récipient, on utilise comme récipient une bouteille pourvue d'un tube plongeur et on introduit la charge de $CO_2$ par le tube plongeur puis, la bouteille étant disposée tête en bas et après une période de stabilisation du $CO_2$, on introduit la charge d'hélium par le tube plongeur, puis on dispose la bouteille tête en haut pour effectuer le soutirage du $CO_2$.

Suivant un autre mode de mise en oeuvre de l'invention, on met la partie supérieure du récipient en communication avec un récipient auxiliaire contenant de l'hélium de manière à fournir de l'hélium au premier récipient sous une pression supérieure à 135 bars environ, et l'on maintient cette communication pendant le soutirage du $CO_2$.

L'invention a également pour objet un équipement destiné à la fourniture d'anhydride carbonique supercritique. Cet équipement comprend un récipient pourvu de moyens de soutirage de liquide et contenant une charge qui se présente sous la forme d'une masse de $CO_2$ supercritique contenant 3 à 6% d'hélium dissous, surmontée d'un ciel gazeux constitué d'hélium sous une pression supérieure à 135 bars environ à la température ambiante.

Dans un mode de réalisation particulier, la partie supérieure du récipient communique avec un récipient auxiliaire contenant de l'hélium sous une pression supérieure à 135 bars environ.

Des exemples de mise en oeuvre de l'invention vont maintenant être décrits en regard des dessins annexés, sur lesquels :

– les figures 1 et 2 illustrent deux variantes du procédé suivant l'invention ;

– la figure 3 est en vue en élévation, partiellement en coupe, d'un équipement conforme à l'invention ; et

– la figure 4 représente en coupe longitudinale un détail de l'équipement de la figure 3.

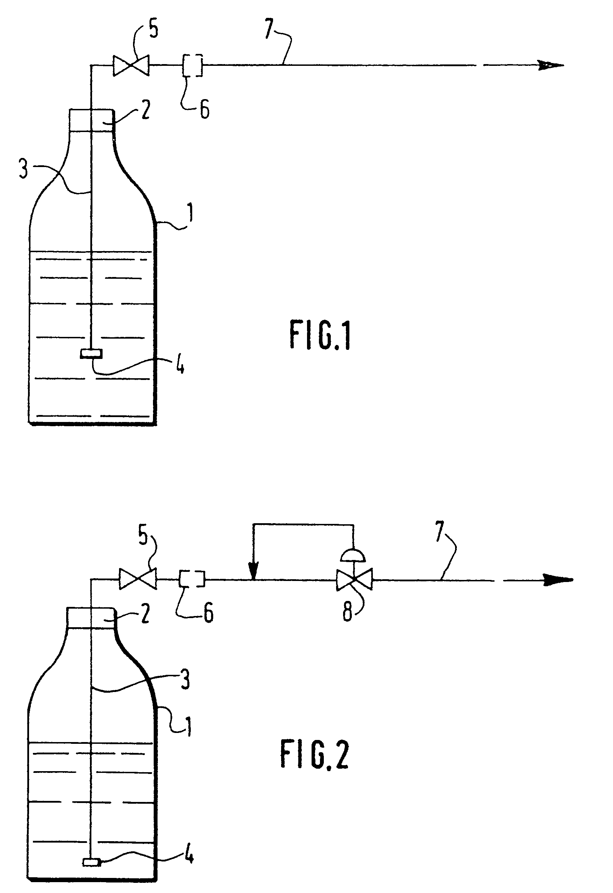

On a représenté à la figure 1, en coupe longitudinale, une bouteille 1 en acier dont le col est équipé d'un bouton 2 traversé par un tube plongeur 3, lequel comporte à son extrémité inférieure un orifice calibré 4. Le tube 3 descend jusqu'à un niveau situé nettement au-dessus du fond de la bouteille et qui sera défini plus loin.

A l'extérieur de la bouteille, le tube 3 est pourvu d'un robinet d'arrêt 5 et se termine par un raccord 6 pour le raccordement d'une conduite d'utilisation 7.

La bouteille 1 est remplie de la façon suivante.

Après avoir effectué un traitement préalable classique pour les bouteilles destinées à recevoir des gaz très purs, on introduit dans la bouteille par le tube 3 une charge de $CO_2$ liquide en provenance d'un stockage à –20°C, 20 bars, au moyen d'une pompe de circulation et d'un réchauffeur qui l'amène aux environs de 0°C, ce pour des raisons liées à la résilience de l'acier dont est constituée la bouteille 1. Le $CO_2$ a une pureté de 99,998% et, par pesée de la bouteille, on en mesure une quantité donnée. Cette quantité, qui doit être suffisante pour que l'orifice calibré 4 soit immergé, correspond par exemple à un volume de liquide égal à la moitié du volume de la bouteille.

On laisse ensuite la bouteille revenir à la température ambiante, ce qui constitue une phase de stabilisation du $CO_2$ qui dure au moins deux heures.

Puis on dispose la bouteille tête en bas, par tout moyen de manipulation approprié. L'orifice calibré 4 émerge alors du liquide, et on attend, pendant une nouvelle phase de stabilisation de l'ordre de 10 mn, que le liquide se soit bien rassemblé et que l'équilibre liquide-vapeur soit de nouveau atteint.

On introduit alors par le tube 3 une charge d'hélium à haute pureté (moins de 5 ppm d'eau, moins de 5 ppm d'oxygène), jusqu'à atteindre une pression de 160 bars environ. Cette pression est choisie aussi haute que possible mais telle que, pour la température ambiante maximale prévisible (par exemple pour +50°C), elle reste au-dessous de la pression de service de la bouteille 1.

Enfin, on remet la bouteille tête en haut et, après un nouveau temps de stabilisation de l'ordre de une heure, elle est prête à fournir du $CO_2$ supercritique par simple ouverture du robinet 5.

La bouteille se trouve alors dans l'état représenté à la figure 1, c'est-à-dire qu'elle contient une charge constituée :

– d'une masse de $CO_2$ liquide supercritique contenant une certaine quantité d'hélium dissous, et

– d'un ciel gazeux sous 160 bars environ, constitué uniquement d'hélium.

Les analyses ont montré que lorsque la pression de l'hélium décroît de 160 bars à 135 bars environ, la teneur en hélium dissous du $CO_2$ liquide est faible et se maintient à une valeur sensiblement constante comprise entre 3 et 4%, alors que cette teneur croît brusquement lorsque la pression descend au-dessous de 135 bars environ. Par conséquent, on arrêtera le soutirage lorsque la pression, qui décroît au fur et à mesure de la baisse du niveau du liquide, atteint une valeur limite non inférieure à cette valeur de 135 bars environ.

Pour cela, on peut, comme représenté à la figure 1, ne faire descendre le tube 3 que jusqu'à un niveau correspondant au volume de ciel gazeux qui fournit la pression de 135 bars environ. Si le volume de remplissage du $CO_2$ liquide est choisi à l'avance, par exemple égal à peu près à la moitié de la capacité de la bouteille comme indiqué plus haut, on en déduit par calcul le niveau en question. En variante, si l'on préfère soutirer la quasi-totalité du liquide, le tube 3 descendra presque jusqu'au fond de la bouteille, et l'on calculera le volume initial du $CO_2$ liquide de façon correspondante.

Cependant, dans ces deux cas, la conduite 7 véhiculera un mélange gazeux $CO_2$-hélium, ou de l'hélium pur, en fin de soutirage. Pour éviter ceci, on peut faire appel au mode de mise en oeuvre illustré à la figure 2 : la bouteille contenant initialement à peu près la moitié de son volume en $CO_2$ liquide, le tube 3 descend presque jusqu'au fond de la bouteille, et la conduite 7 d'utilisation est équipée d'une vanne pressostatique 8 qui se ferme lorsque la pression descend à la valeur limite de 135 bars environ. Le soutirage s'arrête ainsi automatiquement, et l'on change alors de bouteille.

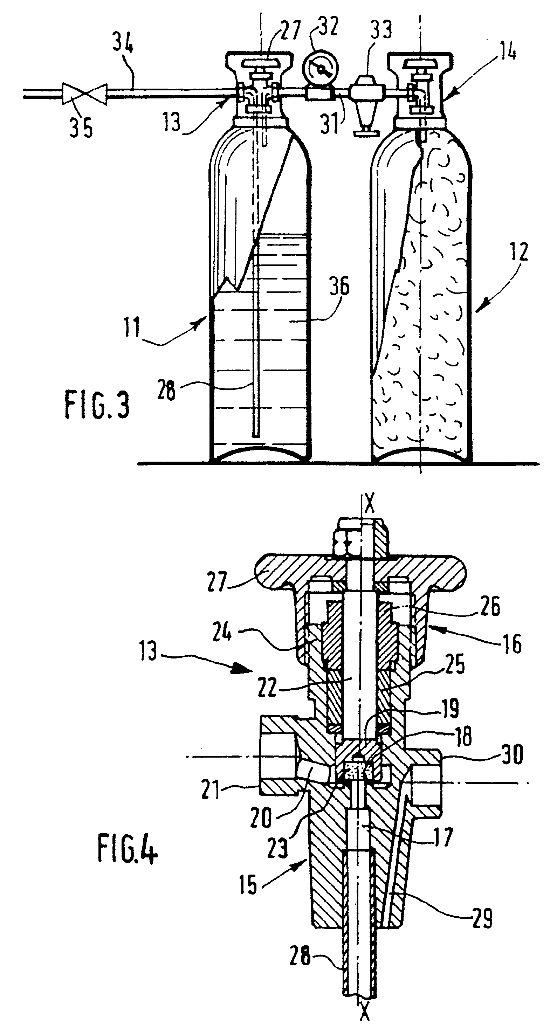

On a représenté à la figure 3 un équipement constitué de deux bouteilles 11 et 12 de mêmes dimensions équipées chacune d'un robinet 13, 14 respectivement.

Le robinet 13, représenté à plus grande échelle à la figure 4, comprend un corps 15, d'axe général X-X vertical, et un dispositif de manoeuvre 16. La partie inférieure du corps 15, fixée dans le col de la bouteille 11, définit un conduit d'entrée 17 qui se termine par un siège 18 et débouche dans une chambre 19. Un conduit de soutirage 20, qui se termine par un raccord principal 21, part latéralement de la chambre 19, et celle-ci présente un orifice supérieur dans lequel coulisse l'extrémité inférieure dela tie de manoeuvre 22 du robinet, laquelle est pourvue d'un clapet 23 pouvant s'appliquer sur le siège 18.

La partie supérieure du corps 15 forme une tubulure 24 qui entoure la tige 22. Le dispositif de manoeuvre 16 comprend, outre la tige 22, un joint d'étanchéité 25 qui entoure cette tige et qui est comprimé dans la tubulaire 24 par un écrou de presse-étoupe 26, ainsi qu'un volant de manoeuvre 27 vissé sur l'extrémité supérieure de la tubulure 24.

Un tube plongeur 28, qui s'étend presque jusqu'au fond de la bouteille 11, est emmanché dans le conduit d'entrée 17, et la partie inférieure du corps 15 comprend un perçage supplémentaire 29 qui met la partie supérieure de la bouteille en communication avec un raccord auxiliaire latéral 30.

Le robinet 14 est identique au robinet 13, à ceci près que son corps est dépourvu du perçage 29 et du raccord auxiliaire 30. De plus, la bouteille 12 ne comporte pas de tube plongeur, de sorte que le conduit d'entrée 17 du robinet 14 débouche directement dans la partie supérieure de cette bouteille.

Comme on le voit à la figure 3, le raccord 30 du robinet 13 est relié au raccord 21 du robinet 14 par une conduite 31 équipée d'un manomètre 32 et d'un détendeur 33. De plus, une conduite d'utilisation 34 munie d'une vanne d'arrêt 35 est reliée au raccord 21 de la bouteille 11.

Au départ, la bouteille 12 contient de l'hélium sous une pression nettement supérieure à 135 bars, par exemple sous 200 bars ; le robinet 14 est fermé, et la bouteille 11 est remplie jusqu'à un niveau prédéterminé de $CO_2$ liquide, de la manière décrite en regard des figures 1 et 2, à travers la conduite 34.

Puis, la vanne 35 étant fermée, on règle le détendeur 33 sur une pression de sortie $\underline{P}$ au moins égale à 135 bars environ, et l'on ouvre le robinet 14 de la bouteille 12. Le $CO_2$ liquide est ainsi pressurisé à la pression $\underline{P}$ et se trouve à l'état supercritique. La bouteille 11 contient alors une masse 36 de $CO_2$ liquide supercritique contenant environ 3 à 6% d'hélium dissous, surmontée d'un ciel gazeux constitué uniquement d'hélium.

On peut alors soutirer le $CO_2$ supercritique par la conduite 34, en ouvrant la vanne 35. Au fur et à mesure de la baisse du niveau du $CO_2$ liquide, de l'hélium passe de la bouteille 12 à la bouteille 11, et celle-ci reste constamment à la même pression $\underline{P}$. Ceci reste vrai tant que la pression de la bouteille $\overline{12}$ reste supérieure à la valeur $\underline{P}$ ; par suite, en choisissant convenablement le niveau initial du $CO_2$ liquide dans la bouteille 11, on peut soutirer la totalité du $CO_2$ sous cette même pression $\underline{P}$.

En variante, on pourrait se passer du détendeur 33. Le $CO_2$ serait alors délivré sous une pression progressivement décroissante à partir de la pression initiale régnant dans la bouteille 12.

## Revendications

1. Procédé pour fournir de l'anhydride carbonique supercritique, dans lequel on introduit une charge de $CO_2$ liquide dans un récipient (1 ; 11) résistant à la pression pourvu de moyens (3 ; 28) de soutirage de liquide, puis on introduit dans ce récipient une charge d'hélium, et on soutire le $CO_2$ par lesdits moyens de soutirage de liquide, caractérisé en ce qu'on introduit l'hélium jusqu'à une pression supérieure à 135 bars environ à la température ambiante, et on arrête le soutirage du $CO_2$ lorsque la pression est descendue à une valeur limite non inférieure à 135 bars environ à la température ambiante.

2. Procédé suivant la revendication 1, caractérisé en ce que, pour limiter la pression de soutirage, on soutire le $CO_2$ au moyen d'un tube plongeur (3), le volume de la charge de $CO_2$ et la longueur de ce tube étant choisis de façon que la pression de l'hélium ne descende pas au-dessous de 135 bars environ en fin de soutirage du $CO_2$.

3. Procédé suivant la revendication 1, caractérisé en ce qu'on soutire le $CO_2$ liquide à travers une conduite (7) munie d'une vanne pressostatique (8)

4. Procédé suivant l'une quelconque des revendications 1 à 3, caractérisé en ce que le récipient (1) est une bouteille pourvue d'un tube plongeur (3) et en ce qu'on introduit la charge ce $CO_2$ par le tube plongeur puis, la bouteille étant disposée tête en bas et après une période de stabilisation du $CO_2$, on introduit la charge d'hélium par le tube plongeur, puis on dispose la bouteille tête en haut pour effectuer le soutirage du $CO_2$.

5. Procédé suivant la revendication 1, caractérisé en ce qu'on met la partie supérieure dudit récipient (11) en communication avec un récipient auxiliaire (12) contenant de l'hélium de manière à fournir de l'hélium au premier récipient (11) sous uni pression supérieure à 135 bars environ, et l'on maintient cette communication pendant le soutirage du $CO_2$.

6. Procédé suivant la revendication 5, caractérisé en ce qu'on détend l'hélium à une pression prédéterminée supérieure à 135 bars environ entre le récipient auxiliaire (12) et le récipient (11) contenant le $CO_2$.

7. Equipement pour la fourniture d'anhydride carbonique supercritique, du type comprenant un récipient (1 ; 11) pourvu de moyens (3 ; 18) ce soutirage de liquide et contenant une charge qui se présente sous la forme d'une masse de $CO_2$ supercritique surmontée d'un ciel gazeux constitué d'hélium, caractérisé en ce que le $CO_2$ contient 3 à 6%. environ d'hélium dissous, l'hélium étant sous une pression supérieure 135 bars environ à la température ambiante.

8. Equipement suivant la revendication 7, caractérisé en ce que la pression de l'hélium à +20°C est d'environ 160 bars.

9. Equipement suivant l'une des revendications 7 et 8, caractérisé en ce que la partie supérieure du récipient (11) communique avec un récipient auxiliaire (12) contenant de l'hélium sous une pression supérieure à 135 bars environ.

10. Equipement suivant la revendication 9, caractérisé en ce que les deux récipients (11, 12) sont reliés par une conduite (31) équipée d'un detendeur (33).


**Ansprüche**

1. Verfahren zum Liefern von superkritischem Kohlendioxid, bei dem man eine Menge von flüssigem $CO_2$ in einen Behälter (1 ; 11) einführt, der den Druck aushält und mit Mitteln (3 ; 28) zur Flüssigkeitsentnahme versehen ist, dann in diesen Behälter eine Menge Helium einführt und das $CO_2$ mit diesen Mitteln zur Flüssigkeitsentnahme entnimmt, **dadurch gekennzeichnet,** daß man das Helium bis zu einem Druck oberhalb etwa 135 bar bei der Umgebungstemperatur einführt und die $CO_2$-Entnahme anhält, wenn der Druck auf einen Grenzwert nicht unterhalb etwa 135 bar bei der Umgebungstemperatur gesunken ist.

2. Verfahren nach Anspruch 1, **dadurch gekennzeichnet,** daß man zur Begrenzung des Entnahmedruckes das $CO_2$ mit Hilfe eines Tauchrohres (3) entnimmt, wobei das Volumen der $CO_2$-Menge und die Länge dieses Rohres derart ausgewählt werden, daß der Druck des Heliums nicht unter etwa 135 bar am Ende der $CO_2$-Entnahme absinkt.

3. Verfahren nach Anspruch 1, **dadurch gekennzeichnet,** daß man das flüssige $CO_2$ über eine Leitung (7) entnimmt, die mit einem pressostatischen Ventil (8) versehen ist.

4. Verfahren nach einem der Ansprüche 1 bis 3, **dadurch gekennzeichnet,** daß der Behälter (1) eine mit einem Tauchrohr (3) versehene Flasche ist und daß man die $CO_2$-Menge durch das Tauchrohr entnimmt, dann, während die Flasche mit dem Kopf nach unten angeordnet ist, und nach einer $CO_2$-Stabilisierungsperiode eine Heliummenge durch das Tauchrohr einführt und sodann die Flasche mit dem Kopf nach oben anordnet, um die $CO_2$-Entnahme durchzuführen.

5. Verfahren nach Anspruch 1, **dadurch gekennzeichnet,** daß man den oberen Teil des Behälters (11) mit einem das Helium enthaltenden Hilfsbehälter derart in Verbindung bringt, daß man das Helium dem ersten Behälter (11) unter einem Druck oberhalb etwa 135 bar zuführt, und daß man diese Verbindung während der $CO_2$-Entnahme aufrechterhält.

6. Verfahren nach Anspruch 5, **dadurch gekennzeichnet,** daß man das Helium zwischen dem Hilfsbehälter (12) und dem das $CO_2$ enthaltenden Behälter (11) auf einen vorbestimmten Druck oberhalb etwa 135 bar entlastet.

7. Vorrichtung zum Liefern von superkritischem Kohlendioxid mit einem Behälter (1 ; 11), der mit Mitteln (3 ; 18) zur Flüssigkeitsentnahme versehen ist und eine Beschickung enthält, die in der Form einer Masse von superkritischem $CO_2$ vorliegt, die von einer aus Helium bestehenden Gasatmosphäre überlagert ist, **dadurch gekennzeichnet,** daß das $CO_2$ etwa 3 bis 6% gelöstes Helium enthält, wobei das Helium sich unter einem Druck oberhalb etwa 135 bar bei der Umgebungstemperatur befindet.

8. Vorrichtung nach Anspruch 7, **dadurch gekennzeichnet,** daß der Druck des Heliums bei 20°C etwa 160 bar beträgt.

9. Vorrichtung nach einem der Ansprüche 7 und 8, **dadurch gekennzeichnet,** daß der obere Teil des Behälters (11) mit einem Hilfsbehälter (12) in Verbindung steht, der das Helium unter einem Druck oberhalb etwa 135 bar enthält.

10. Vorrichtung nach Anspruch 9, **dadurch gekennzeichnet,** daß die beiden Behälter (11, 12) durch eine Leitung (31) verbunden sind, welche mit einem Druckminderventil (33) ausgestattet ist.

## Claims

1. A process for supplying supercritical carbon dioxide, in which a charge of liquid $CO_2$ is fed into a pressure-resistant container (1 ; 11) provided with means (3 ; 28) for drawing off liquid, then a charge of helium is fed into the same container and the $CO_2$ is drawn off by the said means for drawing off liquid, characterised in that the helium is fed in to a pressure greater than approximately 135 bars at ambient temperature, and the drawing off of $CO_2$ is stopped when the pressure has fallen to a limiting value not less than approximately 135 bars at ambient temperature.

2. A process according to Claim 1, characterised in that, in order to limit the drawing-off pressure, the $CO_2$ is drawn off by means of a plunger tube (3), the volume of $CO_2$ and the length of the tube being chosen such that the helium pressure does not fall below approximately 135 bars after the $CO_2$ is drawn off.

3. A process according to Claim 1, characterised in that the liquid $CO_2$ is drawn off through a pipe (7) fitted with a pressure-sensitive valve (8).

4. A process according to any of Claims 1 to 3, characterised in that the container (1) is a bottle fitted with a plunger tube (3) and in that the charge of $CO_2$ is fed in through the plunger tube then, the bottle having been stood upside down and after a period for the $CO_2$ to stabilise, the charge of helium is fed in through the plunger tube, then the bottle is stood upright in order to draw off the $CO_2$.

5. A process according to Claim 1, characterised in that the upper part of the said container (11) is connected to an ancillary container (12) containing helium so as to supply helium to the first container (11) under a pressure greater than approximately 135 bars, and that this connection is maintained while the $CO_2$ is drawn off.

6. A process according to Claim 5, characterised in that the helium pressure is decreased to a predetermined value greater than approximately 135 bars between the ancillary container (12) and the container (11) of $CO_2$.

7. Equipment for supplying supercritical carbon dioxide, of the type comprising a container (1 ; 11) provided with means (3 ; 18) for drawing off liquid and containing a charge in the form of a mass of supercritical $CO_2$ surmounted by a gaseous roof consisting of helium, characterised in that the $CO_2$ contains approximately 3 to 6% dissolved helium, the helium being at a pressure greater than approximately 135 bars at ambient temperature.

8. Equipment according to Claim 7, characterised in that the pressure of the helium at +20°C is around 160 bars.

9. Equipment according to either of Claims 7 or 8, characterised in that the upper part of the container (11) is connected to an ancillary container (12) containing helium at a pressure greater than approximately 135 bars.

10. Equipment according to Claim 9, characterised in that the two containers (11 ; 12) are connected by a pique (31) fitted with a pressure reducing valve (33).

FIG.1

FIG.2

FIG.3

FIG.4